# FASCICULE DE BREVET EUROPEEN

(11) **EP 2 389 903 B1**
(45) Date de publication et mention de la délivrance du brevet: **17.06.2015**
(21) Numéro de dépôt: 11167529.4
(22) Date de dépôt: 25.05.2011
(51) Int. Cl.: A61F 2/44

(54) **Système pour réaliser une arthrodèse entre deux vertèbres et ancillaires de pose**
System zur Durchführung einer Arthrodese zwischen zwei Wirbeln, und Zubehörteile zur Durchführung
System for performing arthrodesis between two vertebrae and installation accessories

(30) Priorité: 25.05.2010 FR 1002189
(43) Date de publication de la demande: 30.11.2011
(73) Titulaire: Prosteel, 31521 Ramonville Cedex (FR); Minelli, Nadia, 31520 Ramonville Saint Agne (FR); Gille, Olivier, 33600 Pessac (FR); Renaud, Christian, 81160 Arthes (FR); Limito, Jean-François, 31130 Balma (FR)
(72) Inventeur: Gille, Olivier, 33600 Pessac (FR); Renaud, Christian, 81160 Arthes (FR)
(74) Mandataire: Hartmann, Jean-Luc

(56) Documents cités:
- WO-A1-98/48738
- WO-A1-02/067786
- WO-A1-2004/069106
- WO-A2-02/058593
- US-A1- 2008 249 569
- US-B1- 6 629 998

## Description

### Domaine technique

La présente invention concerne les systèmes pour réaliser une arthrodèse entre deux vertèbres consécutives notamment au moyen de cages intersomatiques aptes à être intercalées entre les deux vertèbres, plus particulièrement dans le cas de vertèbres cervicales.

### État de la technique antérieure.

Il est connu que, dans le cas de certains traumatismes subis par une colonne vertébrale, les praticiens sont amenés à effectuer deux sortes d'interventions chirurgicales.

Quand le traumatisme ne concerne qu'un disque intervertébral et que les deux vertèbres consécutives encadrant ce disque sont en bon état, le praticien peut opter pour le remplacement du disque par une prothèse dont la fonctionnalité est sensiblement équivalente à celle du disque originel, c'est-à-dire laissant aux deux vertèbres la possibilité de se déplacer l'une par rapport à l'autre dans des mouvements de rotation et/ou de translation.

En revanche, quand le traumatisme est plus important, le praticien peut choisir de solidariser les deux vertèbres consécutives par arthrodèse. Pour ce faire, le disque intervertébral est retiré et remplacé par une cage dite "intervertébrale" associée généralement à un élément favorisant la fusion des éléments arthrodèsés, par exemple un greffon osseux.

Le mode opératoire pour l'implantation de la cage est bien connu des praticiens, mais il se pose malgré tout, des problèmes pour stabiliser la cage avec les corps osseux des deux vertèbres, essentiellement pour que la cage ne se déplace pas accidentellement après qu'elle ait été implantée. Notamment on cherche à écarter tout risque de mobilisation antérieure secondaire de la cage.

Les moyens les plus courants existant actuellement sont constitués par une plaque qui est fixée sur la cage et respectivement sur les deux corps osseux des vertèbres au moyen de vis ou analogues. Cette réalisation donne de bons résultats.

On connaît aussi des cages de fusion aptes à être insérées entre des corps vertébraux et être fixées à ces derniers par des éléments d'ancrage sous forme de pion ou de vis. Typiquement ces éléments d'ancrage présentent chacun une fête et une tige en pointe et sont engagés par leur tête dans des perçages traversant formés dans l'une des parois de la cage. Par leur tige en pointe ces éléments d'ancrage sont enfichés dans le corps vertébral adjacent. De tels modes de réalisation sont connus notamment du brevet européen EP302719 qui montre une cage en fer à cheval, prévue pour recevoir un substitut osseux et dotée d'une série de perçages orientés débouchant chacun d'une part dans la face incurvée externe de la forme en fer-à-cheval dans l'une des faces inférieure ou supérieure, chaque perçage étant pourvu d'un épaulement d'appui de la tête d'une vis de fixation de la cage au corps vertébral adjacent. Les perçages débouchent en alternance dans la face supérieure et dans la face inférieure.

L'inconvénient de telles cages de fusion réside essentiellement dans le fait que la partie interne à la cage de l'élément d'ancrage est isolée du corps vertébral adjacent et ne s'ancre pas dans ce corps vertébral. Ainsi sous certaines conditions, l'élément d'ancrage en raison d'un ancrage insuffsant peut s'extraire du corps osseux.

On connaît du WO 02/058593 un implant intervertébral comprenant une cage dotée de plusieurs perçages prévus pour recevoir des broches osseuses. Les perçages sont formés de manière à n'entourer que partiellement l'extrémité arrière des broches qu'ils reçoivent de façon que les extrémités arrière puisse former saillie sur les surfaces de contact de la cage avec les corps vertébraux adjacents.

On connaît du document US 2008/0249569 un implant intervertébral doté de perçages prévus is pour recevoir des vis de fixation aux corps osseux adjacents. Les entrées de ces perçages, située sur la face antérieure de l'implant, sont recouvertes par une plaque de rétention antérieure.

On connaît du WO 02/067786 un instrument d'insertion prévu pour quidage des instruments chirurgicaux et l'insertion d'implants entre deux vertèbres adjacentes. Ce dispositif possède un écarteur prévu pour être disposé entre deux vertèbres adjacentes et un élément s'étendant en arrière de l'écarteur définissant un canon de guidage d'un instrument chirurgical.

La présente invention à pour but de réaliser un système pour réaliser une arthrodèse entre deux vertèbres consécutives, plus particulièrement des vertèbres cervicales, dont la structure pallie en grande partie les inconvénients des systèmes de l'art antérieur et notamment les problèmes de migration antérieure secondaire des broches ou vis d'ancrage dans les corps vertébraux.

### Exposé de l'invention.

La présente invention a pour objet un système pour réaliser une arthrodèse entre deux première et seconde vertèbres consécutives, plus particulièrement des vertèbres cervicales, comportant :
- une cage apte à être interposée entre les deux dites vertèbres en remplacement du disque intervertébral qui était originellement situé entre ces deux vertèbres, ladite cage étant définie dans une enveloppe parallélépipédique rectangle, tacite cage comportant deux première et seconde faces d'appui, opposées, aptes à venir au contact respectivement des deux corps osseux des deux dites vertèbres quand la cage est interposée entre elles et une paroi latérale réunissant les deux dites faces opposées, ladite paroi latérale étant composée de quatre portions de paroi à savoir une portion antérieure (13a) et une portion postérieure (13b) normales au plan sagittal et deux portions (13c) gauche et droite, et
- des moyens pour stabiliser ladite cage avec le corps osseux d'au moins l'une première des deux vertèbres consécutives, ces dits moyens étant des moyens de stabilisation postéro-antérieure et étant constitués par deux ensembles ensemble (E1, E2) comportant chacun au moins :
- un élément d'ancrage, sous forme de pion comportant :
   * une tige définie selon un premier axe longitudinal entre une extrémité proximale et une extrémité distale, l'extrémité distale étant conformée en extrémité pénétra ite, ladite tige étant apte à être ancrée dans le corps osseux de la première vertèbre en pénétrant, dans ce corps osseux, par son extrémité distale pénétrante,
   * une tête cylindrique ayant une longueur déterminée définie selon un deuxième axe longitudinal, la dite tête étant solidaire de ladite tige et étant formée en extrémité proximale de cette dernière,
   • et un logement de forme sensiblement cylindrique défini selon un troisième axe longitudinal et réalisé dans ladite cage de façon qu'il débouche sur ladite paroi latérale par un orifice d'entrée et sur la première face d'appui par un orifice de sortie, edit troisième axe longitudinal faisant un angle déterminé par rapport au plan passant par la deuxième face d'appui, ce logement étant doté d'une fente radiale s'étendant d'un orifice à l'autre et débouchant sur la première face d'appui, et ledit logement étant en outre réalisé de façon que, lorsque ladite tête cylindrique est enfichée dans le logement, le premier axe longitudinal de la tige fasse avec le plan de cette première face un angle non nul et que, lorsque ladite cage est interposée entre les deux vertèbres consécutives, ladite tige déborde du logement par l'orifice de sortie et ladite tête par la fente radiale et pénètrent toutes deux dans le corps osseux de la première vertèbre qui est au contact de cette première face,
      - ledit système comportant en en outre une plaque antérieure venant en recouvrement partiel au moins de l'entrée de chaque logement pour empêcher la sortie de la tête cylindrique hors de ce logement après qu'elle y ait été enfichée, et des moyens pour solidariser cette plaque antérieure avec la cage, lesquels sont constitués d'une part, par deux pattes latérales que comporte ladite plaque ces pattes latérales présentant en extrémité des bossages de rétention, et l'autre part par des formes en creux complémentaires de celles des bossages et prévues pour recevoir ces derniers, ces formes en creux étant ménagées dans es parois gauche et\droite de la cage,
         caractérisé essentiellement en ce que :
         les arêtes de la plaque antérieure, des pattes latérales et des bossages sont arrondies, que l'extrémité libre de chaque patte latérale est arrondie, que chaque bossage est cylindrique, que ladite plaque vient en recouvrement partiel des deux entrées des deux logements respectivement des deux ensembles (E1, E2) et que la plaque antérieure est montée sur la cage avec une aptitude de pivotement limité autour de l'axe géométrique défini par les bossages et les formes en creux, cet axe géométrique de pivotement étant parallèle à la face antérieure de la portion de paroi afin que la migration vers la plaque de l'un des pions, se traduise par un effort de poussée de ce pion par sa face terminale antérieure, contre le bord longitudinal correspondant de la face interne de la plaque antérieure, et par voie de conséquence par le basculement de cette plaque antérieure vers l'autre pion et plus particulièrement vers l'entrée du logement accueillant cet autre pion.

Cette disposition, comme on le comprend améliore l'ancrage de la tige au corps osseux adjacent, cet ancrage s'opérant sur toute la longueur de la tige d'ancrage. De plus la disposition de plaque antérieure est de nature à constituer un élément rétentif antérieur supplénientaire pour les éléments d'ancrage afin de prévenir tout risque de mobilisation secondaire de ces derniers. Ces dispositions confèrent à la plaque antérieure une fonction de blocage dynamique permettant, en cas de migration antérieure secondaire des pions 20, de renforcer leur blocage.

Selon une autre caractéristique de l'invention, l'élément d'ancrage est un pion et présente des formes en sapines orientées de telle sorte qu'elles permettent son introduction dans le corps osseux mais s'opposent à son extraction.

Alternativement, selon une autre caractéristique de l'invention, l'élément d'ancrage est une vis.

Selon lune autre caractéristique de l'invention le premier axe de la tige et le deuxième axe de la tête sont confondus, mais en variante, selon une autre caractéristique de l'invention, ces deux axes sont décalés l'un par rapport à l'autre et sont parallèles.

Selon une autre caractéristique de l'invention, le système comporte au moins deux ensembles de stabitisatian postéro-antérieure, les deux logements respectivement des deux ensembles étant réalisés de façon que, lorsque la tête du pion de chaque ensemble est enfichée dans le logement correspondant, les premiers axes longitudinaux des deux, tiges fassent respectivement avec les plans des première et seconde faces un angle (β) non nul et que, lorsque ladite cage est interposée entre les deux vertèbres, les tiges pénètrent respectivement dans le corps osseux des deux vertèbres respectivement au contact des première et seconde faces de la cage.

### Bref exposé des figures et des dessins

D'autres caractéristiques et avantages de la présente invention apparaîtront au cours de la description suivante donnée en regard des dessins annexés à titre illustratif mais nullement limitatif, dans lesquels :
- la figure 1 est une de profil du système en situation entre deux vertèbres adjacentes,
- la figure 2 est une vue en éclaté partiel du système selon l'invention,
- la figure 3 est une vue de dessus du système selon l'invention,
- la figure 4 est une vue en coupe selon la ligne IV - IV de la figure 3,
- la figure 5 est une vue en plan d'un pion d'ancrage,
- la figure 6 est une vue de dessus de la cage du système selon l'invention,
- la figure 7 est une vue en perspective de la cage selon l'invention,
- la figure 8 est une vue d'une autre forme de réalisation du pion d'ancrage selon l'invention,
- la figure 9 est une vue en coupe brisée, selon deux plans parallèles au plan sagittal, illustrant l'aptitude de pivotement limité de la plaque antérieure,
- la figure 10 est une vue en perspective d'un ancillaire de pose d'une cage du système selon l'invention,

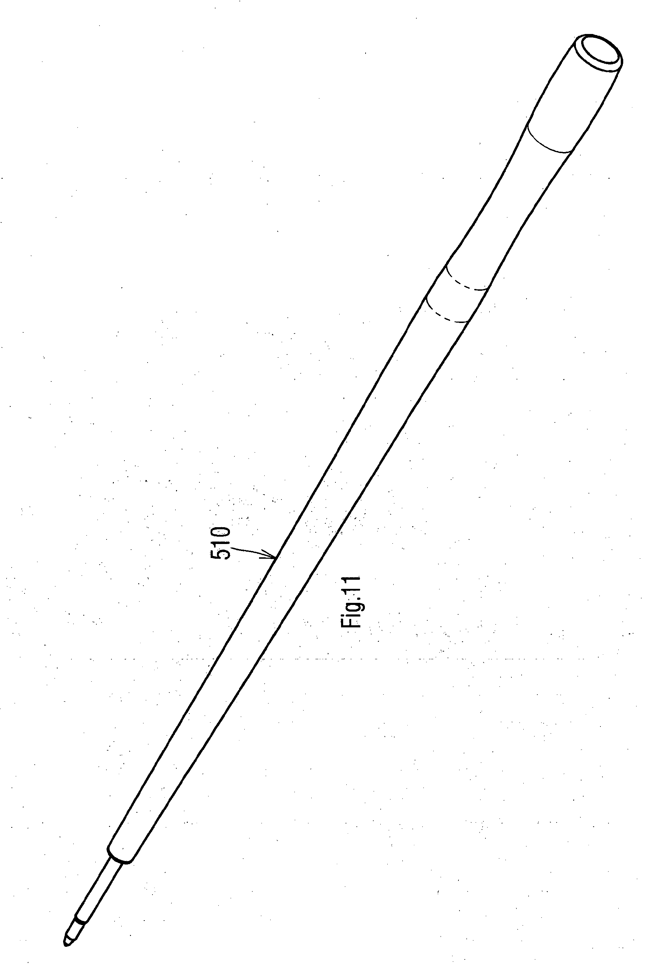

- la figure 11 est une vue en perspective d'un amorçoir,

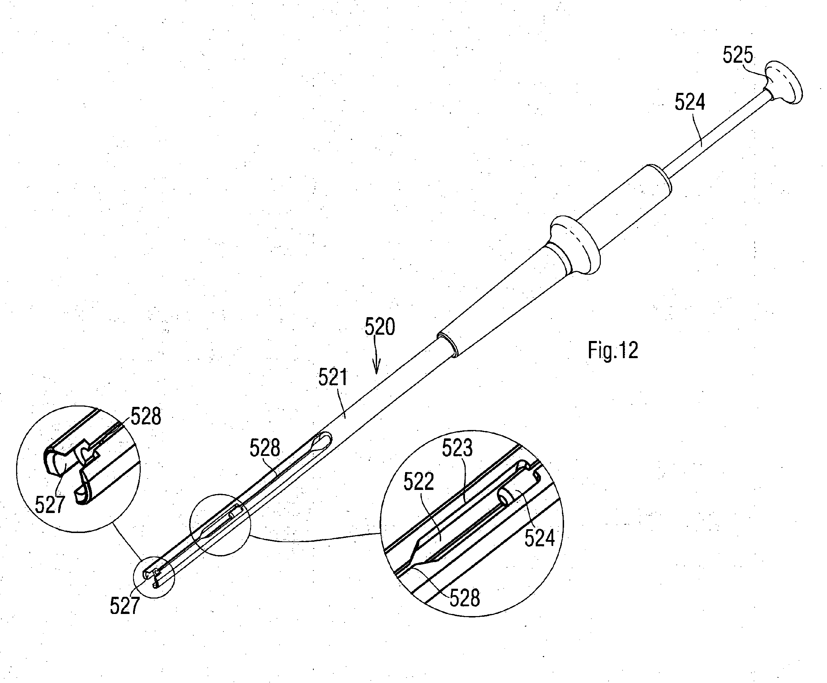

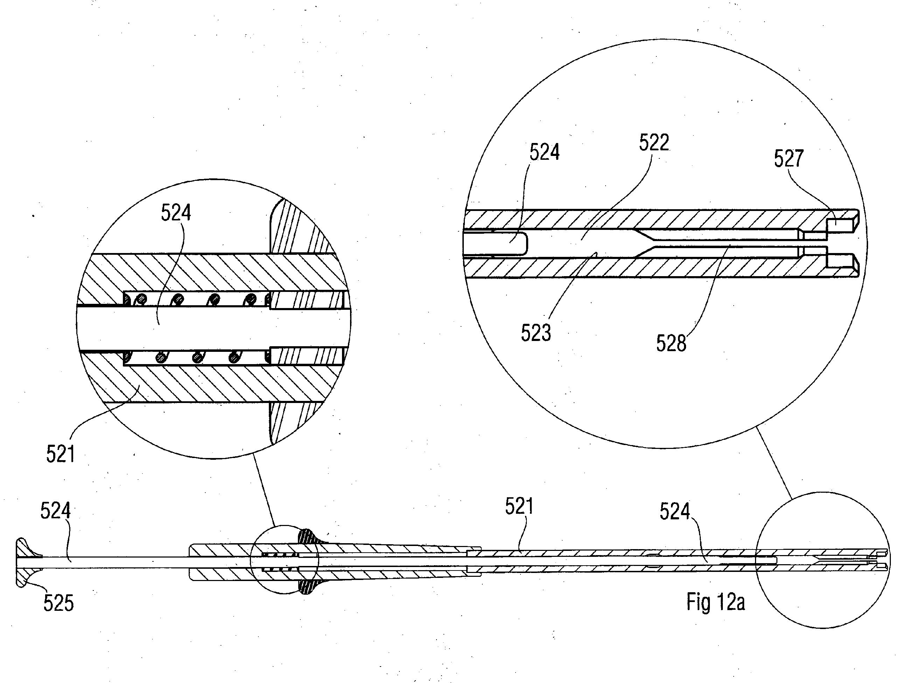

- la figure 12 est une vue en perspective d'un positionneur des pions,
- la figure 12a est une vue en coupe du positionneur selon la figure 12,
- la figure 13 est une vue en perspective d'un ancillaire de pose de la plaque antérieure,

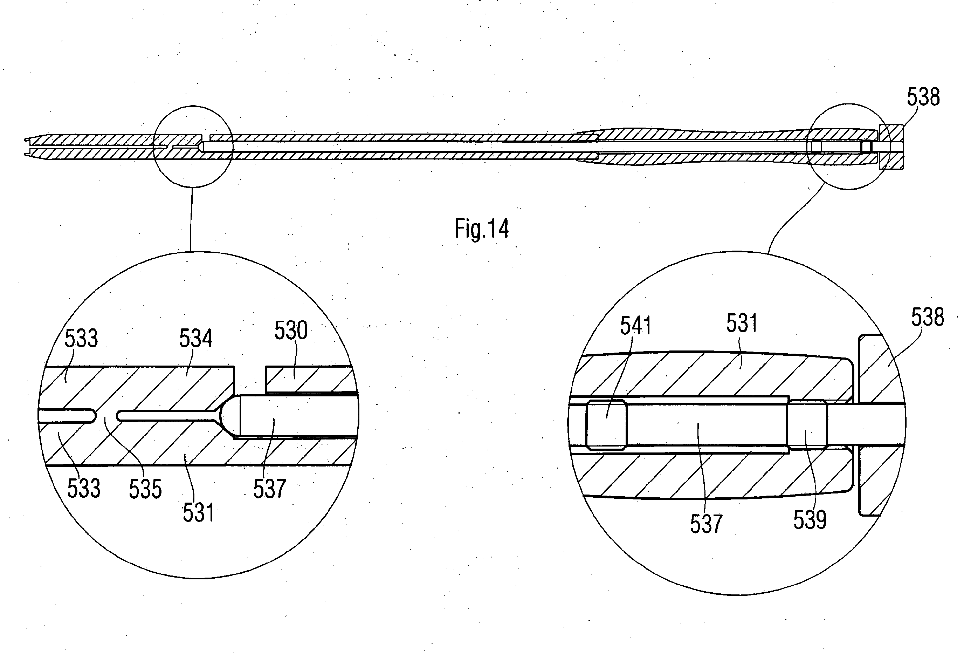

- la figure 14 est une vue en coupe de l'ancillaire de pose de la plaque antérieure.

### Meilleure manière de réaliser l'invention

Il est tout d'abord précisé que, sur les figures, les mêmes références désignent les mêmes éléments, quelle que soit la figure sur laquelle elles apparaissent et quelle que soit la forme de représentation de ces éléments. De même, si des éléments ne sont pas spécifiquement référencés sur l'une des figures, leurs références peuvent être aisément retrouvées en se reportant à une autre figure.

Il est aussi précisé que les figures représentent essentiellement un mode de réalisation de l'objet selon l'invention, mais qu'il peut exister d'autres modes de réalisation qui répondent à la définition de cette invention.

Il est en outre précisé que, lorsque, selon la définition de l'invention, l'objet de l'invention comporte "au moins un" élément ayant une fonction donnée, le mode de réalisation décrit peut comporter plusieurs de ces éléments. Réciproquement, si le mode de réalisation de l'objet selon l'invention tel qu'illustré comporte plusieurs éléments de fonction identique et si, dans la description, il n'est pas spécifié que l'objet selon cette invention doit obligatoirement comporter un nombre particulier de ces éléments, l'objet de l'invention pourra être défini comme comportant "au moins un" de ces éléments.

Il est enfin précisé que lorsque, dans la présente description, une expression définit à elle seule, sans mention particulière spécifique la concernant, un ensemble de caractéristiques structurelles, ces caractéristiques peuvent être prises, pour la définition de l'objet de la protection demandée, quand cela est techniquement possible, soit séparément, soit en combinaison totale et/ou partielle.

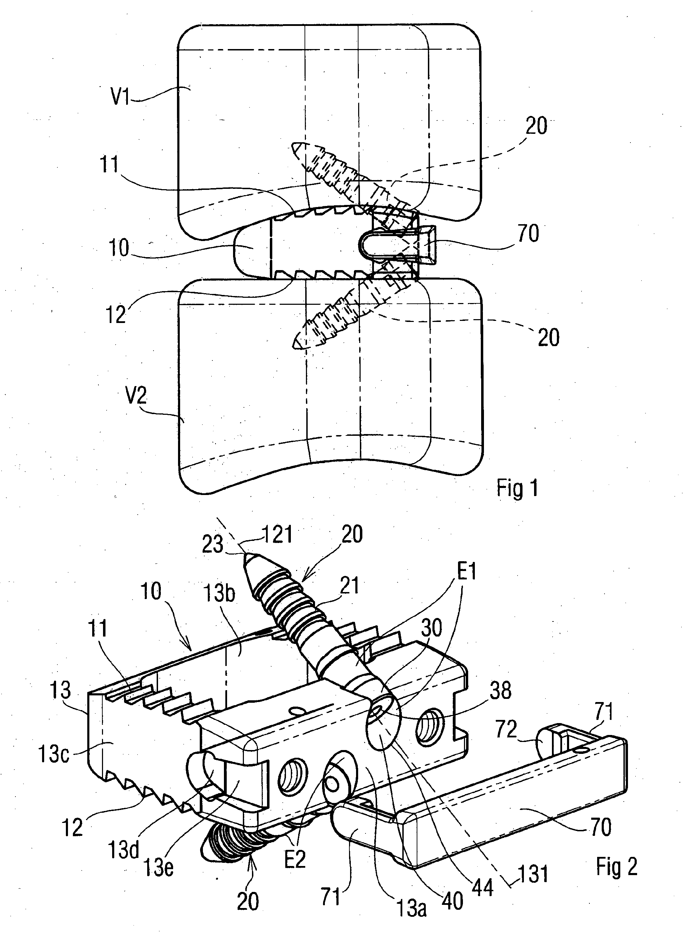

La présente invention concerne un système pour réaliser une arthrodèse entre deux première et seconde vertèbres consécutives V1, V2, figure 1, plus particulièrement, mais non exclusivement, des vertèbres cervicales.

Le système comporte une cage 10, généralement dénommée par les hommes du métier "cage intervertébrale" ou " cage intersomatique", apte à être interposée entre les deux vertèbres en remplacement du disque intervertébral qui était originellement situé entre ces deux vertèbres.

La cage est essentiellement définie dans une enveloppe parallélépipédique rectangle, ou similaire, de façon à comporter deux première et seconde faces opposées 11, 12, d'appui sur les corps vertébraux adjacents et une paroi latérale 13 réunissant ces deux faces opposées et se composant essentiellement de quatre portions de paroi ou assimilables, à savoir une portion antérieure 13a et une portion postérieure 13b normales au plan sagittal et deux portions 13c gauche et droite parallèles au plan sagittal ou inclinées par rapport à ce dernier. Les première et seconde faces opposées 11, 12 sont sensiblement planes ou convexes. Avantageusement la face supérieure est convexe tandis que la face inférieure est plane. Ces deux faces 11, 12 sont aptes à venir au contact respectivement des deux corps osseux des première et seconde vertèbres consécutives V1, V2, (figure 1), quand la cage 10 est interposée entre les deux vertèbres.

Le système selon l'invention comporte en outre des moyens pour stabiliser de manière postéro-antérieure par la cage 10 avec le corps osseux d'au moins l'une première V1 des deux vertèbres consécutives, afin de prévenir une migration postéro-antérieure secondaire de la cage 10, susceptible d'entraîner des dommages au niveau des tissus environnants.

Selon une caractéristique de l'invention, les moyens pour stabiliser de manière postéro-antérieure la cage 10 avec le corps osseux d'au moins l'une première V1 des deux vertèbres consécutives V1, V2, sont constitués par au moins un ensemble E1 comportant un élément d'ancrage 20, sous forme de pion ou de vis, l'élément d'ancrage comportant une tige 21 définie selon un premier axe longitudinal 121 entre une extrémité proximale 22 et une extrémité distale 23, l'extrémité distale étant conformée en extrémité pénétrante, de façon que la tige soit apte à être ancrée dans le corps osseux de la vertèbre V1 en pénétrant dans le corps osseux de cette vertèbre par son extrémité distale pénétrante 23, par impactage ou par vissage selon le cas. L'élément d'ancrage 20 comporte en outre une tête cylindrique 30, de préférence de révolution, ayant une longueur déterminée définie selon un deuxième axe longitudinal 131, ladite tête 30 étant solidaire de l'extrémité proximale 22 de la tige 21.

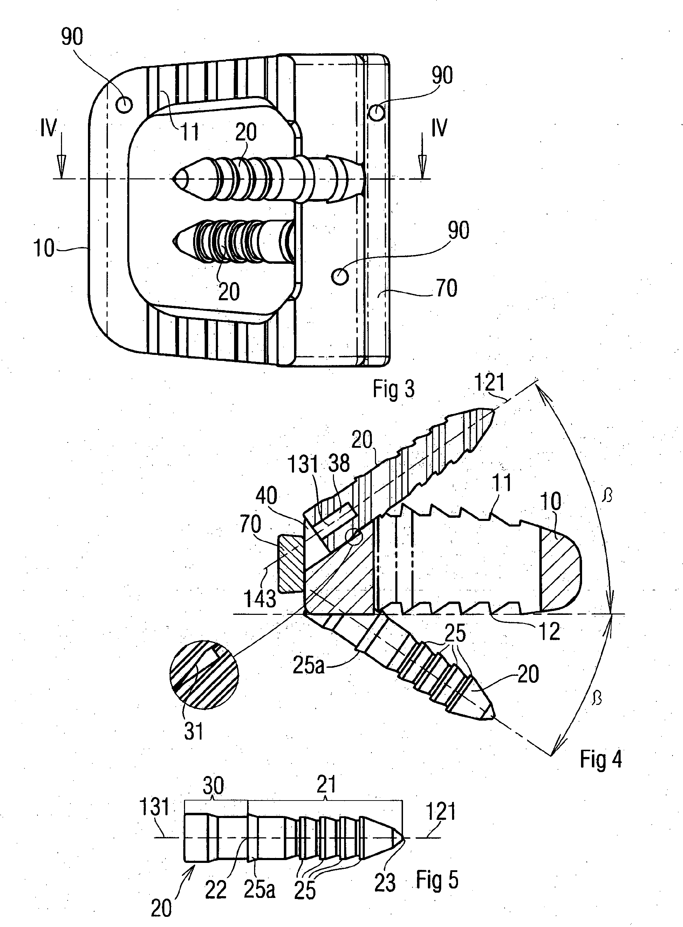

Comme on peut le voir plus particulièrement en figure 5, l'élément d'ancrage est un pion et la tête 30 prolonge axialement la tige 21 leurs axes 121, 131 étant confondus.

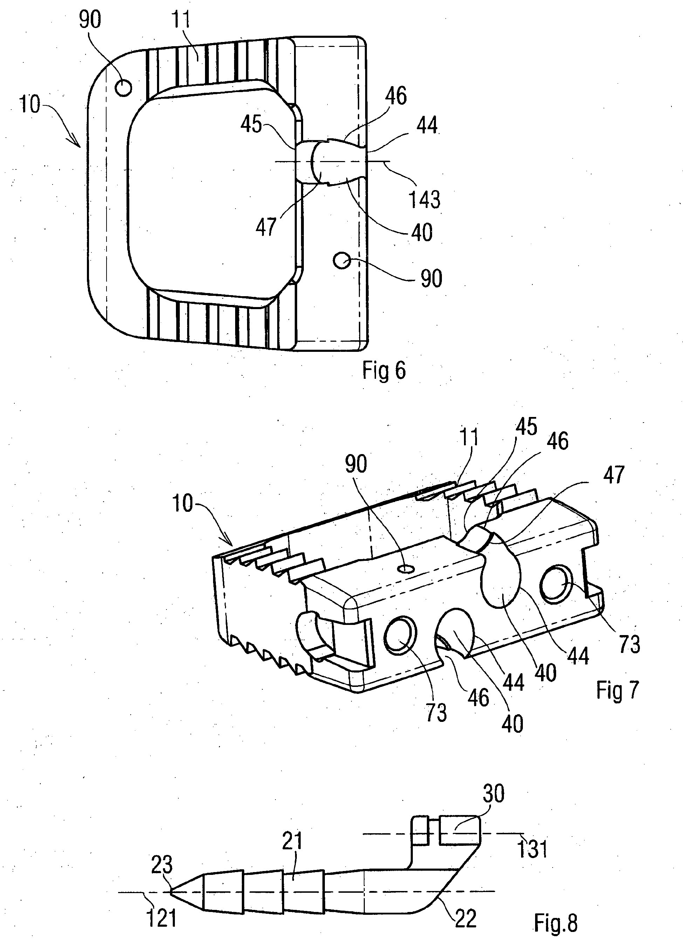

Selon une variante de réalisation, comme représentée en figure 8, l'élément d'ancrage est un pion et la tête 30 est décalée latéralement par rapport à la tige et forme corps avec cette dernière. Dans cette configuration les axes 121 et 131 sont écartés latéralement l'un de l'autre et sont parallèles.

L'élément d'ancrage peut également être constitué par une vis et dans ce cas de figure les axes 121 et 131 seront confondus.

L'ensemble E1 comporte aussi un logement 40 de forme sensiblement cylindrique, défini selon un troisième axe longitudinal 143 et réalisé dans la cage 10 de façon qu'il débouche notamment sur la face antérieure de la portion de paroi 13a par un orifice d'entrée 44, ce troisième axe longitudinal 143 faisant une direction déterminée par rapport au plan passant sensiblement par la seconde face 12 destinée à être en contact avec le corps osseux de la seconde vertèbre V2.

Le logement 40 débouche également sur la première face 11 par un orifice de sortie 45. Ce logement est en outre dotée d'une fente radiale 46 s'étendant de l'orifice d'entrée à l'orifice de sortie, cette fente débouchant dans la face 11.

Le logement 40 a en outre un diamètre au moins égal à celui de la tête cylindrique 30 et est réalisé de façon que, lorsque la tête cylindrique 30 est enfichée dans ce logement 40, le premier axe longitudinal 121 de la tige 21 fasse avec le plan de la seconde face 12 un angle non nul β et que, lorsque la cage 10 est interposée entre les deux vertèbres consécutives V1, V2, la tige 21 pénètre de manière oblique dans le corps osseux de la première vertèbre V1 qui est au contact de la première face 11 et que la tête 30 par sa partie débordante du logement 40 puisse pénétrer dans le corps osseux adjacent.

Selon une réalisation avantageuse et préférentielle, le logement 40 a une profondeur au moins égale à la longueur de la tête cylindrique 30 de façon que la tête cylindrique soit apte à y pénétrer en entier et que, comme illustré sur les figures et dans le but défini ci-après, elle n'émerge en aucune façon de la paroi latérale 13. On peut observer que cette tête 30 cylindrique présente une surface de butée 31, annulaire, adaptée à venir en butée contre un épaulement interne 47 du logement 40. En outre la tige 21 et la tête 30 présentent des formes en sapine 25, 25a, sous forme de collerette annulaire, agencées pour autoriser l'introduction de la tige 21 et de la tête 30 dans le corps osseux et pour s'opposer à leur extraction. De préférence au moins une des formes en sapine 25a de la tige, celle prévue pour être située immédiatement en regard de l'orifice de sortie, présente un diamètre supérieur à celui dudit orifice. Cette disposition après mise en place de l'élément d'ancrage 20 assure la fixation de ce dernier par rapport à la cage 10.

Il est préférable que le troisième axe longitudinal 143, celui de l'orifice 40, fasse, avec le plan de la seconde face 12, un angle égal à l'angle non nul β défini auparavant.

Selon une réalisation avantageuse, le système comporte en outre un trou borgne réalisé dans la tête cylindrique 30 sensiblement suivant le deuxième axe longitudinal 131. Ce trou borgne est prévu pour recevoir un repère radiographique 38.

Le système peut avantageusement comporter en outre une plaque antérieure 70 venant en recouvrement au moins de l'entrée 44 du logement 40 pour empêcher la sortie de la tête cylindrique 30 hors de ce logement 40 après qu'elle y ait été enfichée, et des moyens pour solidariser la plaque antérieure 70 avec la cage 10. Ces moyens peuvent être de tout type, mais préférentiellement ils seront constitués d'une part par deux pattes latérales, élastiques 71 que comporte la dite plaque 70, ces pattes latérales présentant en extrémité des bossages 72 de rétention et d'autre part par des formes en creux 13d complémentaires de celles des bossages et prévues pour recevoir ces derniers, ces formes en creux 13d étant ménagées dans les portions de parois 13c gauche et droite de la cage 10. Avantageusement ces portions de parois 13c présentent chacune une gorge 13e apte à recevoir la patte correspondante 71 de la plaque antérieure 70, cette gorge 13e débouchant radialement dans la forme en creux 13d correspondante. Sous l'effet de l'élasticité des pattes 71, les bossages 72, lorsque la plaque antérieure est en place sur la cage, sont sollicités vers le fond des formes en creux 13d.

Selon la forme préférée de réalisation, les arêtes de la plaque antérieure 70, des pattes latérales 71 et des bossages 72 sont arrondies selon un rayon de courbure approprié afin que lors de la mise en place de la plaque antérieure, tous risques de lésions, par ces arêtes, de l'appareil veineux et des tissus adjacents soient écartés. De plus, pour notamment les mêmes raisons, l'extrémité libre de chaque patte latérale 71 est arrondie et chaque bossage 72 est cylindrique.

Toujours selon la forme préférée de réalisation, la profondeur de chaque gorge 13e est égale ou supérieure à l'épaisseur de chaque patte latérale afin qu'après mise en place de la plaque antérieure 70, les pattes latérales 71 ne soient pas en débordement par rapport aux faces externes à la cage, des parois 13c.

Pour faciliter la mise en place de la plaque antérieure 70, chaque gorge 13e est de forme évasée et s'élargit progressivement depuis la forme en creux 13d correspondante vers la face antérieure de la portion de paroi 13a. Toujours pour faciliter cette introduction, le fond plat de chaque gorge 13e est incliné latéralement vers l'extérieur depuis la face antérieure de la portion de paroi 13a, vers la forme en creux 13d et la face terminale de chaque bossage 72 est oblique par rapport à l'axe géométrique de révolution que possède ce bossage, et est parallèle lors de la mise en place de la plaque antérieure, au fond plat de la gorge correspondante. Lors de cette mise en place, la face terminale de chaque bossage vient glisser sur le fond de la gorge et compte tenu de l'oblicité de ce fond, les pattes élastiques 71 sous l'effet des efforts appliqués sur la face terminale de chaque bossage par le fond de chaque gorge, s'écartent angulairement l'une de l'autre et ce à l'encontre de l'action des forces élastiques internes. Par la suite, lorsque les bossages parviennent au droit de chaque forme en creux, les pattes élastiques 71 sont ramenées l'une vers l'autre sous l'effet des forces élastiques internes et les bossages 72 sont introduits dans les formes en creux 13d.

La face terminale de chaque bossage 72 présente une facette arrière perpendiculaire à l'axe géométrique du dit bossage. De plus, toujours selon la forme préférée de réalisation, chaque bossage présente un méplat antérieur prévu pour venir en regard et contre une face plane de la forme en creux. De telles dispositions maximisent la rétentivité de la plaque 70 dans la cage 10.

Pour obtenir de façon fiable une meilleure stabilisation postéro antérieure de la cage par rapport aux première et seconde vertèbres V1, V2, il est préférable que le système comporte au moins deux ensembles E1, E2, pour stabiliser la cage 10 avec respectivement les deux vertèbres.

Dans ce cas, les deux orifices 40 respectivement des deux ensembles E1, E2 sont réalisés de façon que, lorsque les têtes cylindriques 30 sont enfichées dans les orifices 40, les premiers axes longitudinaux 121 des deux tiges 21 fassent respectivement avec le plan de la seconde face 12 un angle β non nul, les deux angles β n'ayant pas obligatoirement la même valeur bien que cela soit préférable pour simplifier la réalisation industrielle du système et faciliter son implantation, les deux éléments d'ancrage 20 étant alors identiques.

De cette façon, lorsque la cage 10 est interposée entre les deux vertèbres V1, V2, la tige 21 de chaque ensemble pénètre dans le corps osseux de la vertèbre qui est au contact de l'une ou l'autre des première et seconde faces 11, 12 de la cage.

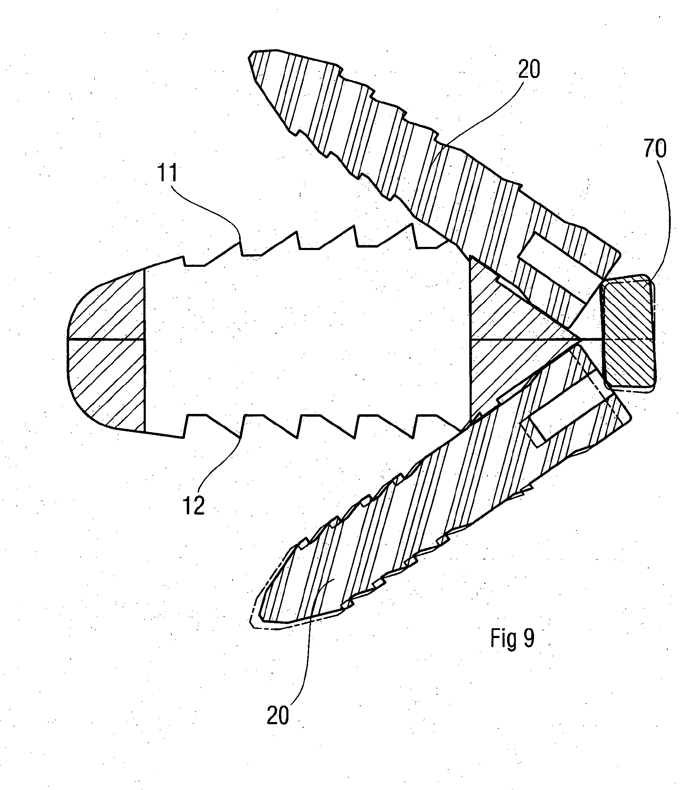

Dans ce cas, il est en outre avantageusement prévu que la plaque antérieure 70 soit conformée pour venir en recouvrement partiel des deux entrées 44 des deux orifices 40 respectivement des deux ensembles E1, E2. Préférentiellement, ce recouvrement n'est que partiel et la plaque antérieure 70, comme on peut le voir en figure 9, est solidarisée à la cage 10 avec une aptitude de pivotement limité autour de l'axe géométrique défini par les bossages 72 et les formes en creux 13d, cet axe géométrique de pivotement étant parallèle à la face antérieure de la portion de paroi 13a. La plaque 70 est ainsi montée avec un léger jeu dans la cage 10 afin de pouvoir pivoter légèrement dans un sens ou dans l'autre autour de l'axe géométrique précité. De préférence, le jeu est limité à deux ou trois dixièmes de millimètres. De telles dispositions confèrent à la plaque antérieure 70 une fonction de blocage dynamique permettant, en cas de migration antérieure secondaire des pions 20, de renforcer leur blocage. En effet sous l'effet de son mouvement migratoire, le pion concerné va venir agir en poussée, par sa face terminale antérieure, contre le bord longitudinal correspondant de la face interne de la plaque antérieure 70, cette face interne étant celle en regard de la face antérieure de la portion de paroi 13a. De cette action, il va en résulter le basculement de cette plaque antérieure 70 vers l'autre pion et plus particulièrement vers l'entrée 44 du logement 40 accueillant cet autre pion.

Selon la forme préférée de réalisation, l'amplitude du pivotement de la plaque antérieure est, à partir d'une position médiale, de cinq degrés vers le haut et de cinq degrés vers le bas. L'amplitude totale est donc de dix degrés.

La cage 10, peut être de différentes formes et avoir différentes structures, comme celles que l'on trouve actuellement sur le marché. Cependant, elle aura, de préférence, une forme et une structure comme celle illustrée en figure 2.

Comme défini précédemment, la cage 10 est inscrite dans un parallélépipède rectangle, avec des faces légèrement convexe pour l'une et plane pour l'autre pour s'adapter aux formes anatomiques des corps vertébraux et/ou pour des raisons de réalisation mécanique.

Elle peut en outre comporter au moins une percée traversante, réalisée entre les deux faces opposées 11, 12, cette percée étant conçue pour recevoir un substitut osseux ou un greffon osseux afin de favoriser, de façon connue en elle-même, l'arthrodèse entre les deux vertèbres V1, V2.

Mais elle peut aussi comporter une (ou plusieurs) percée traversante qui débouche sur sa paroi latérale 13 et de préférence communique avec la percée traversante débouchant sur ses deux faces opposées 11, 12.

En outre, toutes les faces de la cage 10, mais essentiellement les deux faces opposées 11, 12, peuvent comporter des aspérités de toute forme, par exemple des picots ou analogues mais avantageusement des aspérités en dents de scie comme illustré notamment sur les figures 1 à 4 et 6, 7, dans le but de sécuriser encore plus la stabilité de la cage dans les deux corps vertébraux.

La cage 10 peut être réalisée en tout matériau biocompatible, avantageusement en PEEK (sigle anglais d'une matière plastique thermostable connue en français sous le nom de poly-éther-éther-cétone), comme d'ailleurs tous les autres éléments du système selon l'invention, à savoir les éléments d'ancrage 20, la plaque antérieure 70, les pattes 71 et les bossages 72.

De plus, quand la cage est en un matériau comme du PEEK, elle est radio-transparente. Dans ce cas, elle peut avantageusement comporter, en des endroits choisis et bien connus des personnes du métier, des logements 90 aptes à recevoir des inserts en un matériau radio-opaque permettant de vérifier par radiographie la position de la cage 10 par rapport aux vertèbres.

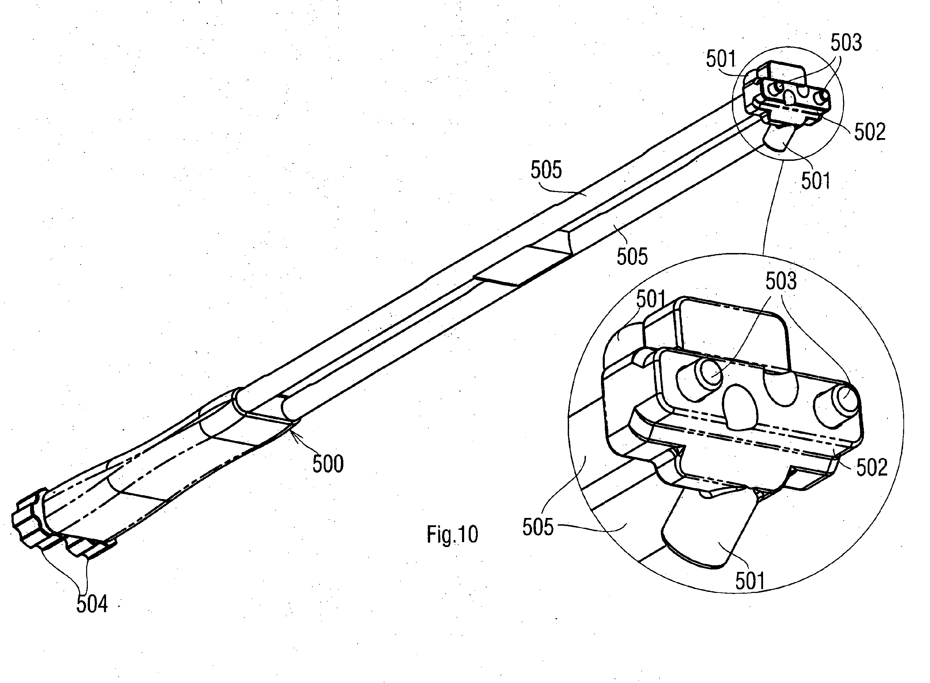

Le système selon l'invention s'implante entre deux vertèbres V1, V2 de la façon suivante à l'aide d'un outil ancillaire 500 tel que représenté en figure 10 adapté qui se fixe à la cage 10 au moyen de deux perçages borgnes 73 pratiqués dans la portion antérieure de paroi 13a de la cage, ces perçages, à cette fin, étant avantageusement taraudés. On peut remarquer que les orifices 44 sont situés dans l'intervalle séparant les deux perçages borgnes 73. Cet ancillaire est formé d'un bâti allongé formant manche de préhension, constitué de deux éléments tubulaires 505 portant en extrémité distale une même platine 502 dotée de deux canons de guidage 501 dont l'orientation est celle des logements 40 dans la cage 10. Chaque éléments tubulaires 502 reçoit une tige cylindrique 503 dont le segment distal déborde de la platine 502 et est fileté pour être engagé en vissage dans l'un des taraudages 73. En vue de sa manoeuvre en vissage et dévissage, chaque tige cylindrique 503 reçoit en extrémité proximale, extérieurement à l'élément tubulaire 505 qui l'accueille, un bouton de manoeuvre 504.

Au moyen de cet ancillaire, le praticien positionne la cage 10 entre les deux vertèbres V1, V2.

Puis, tout en maintenant l'ancillaire 500 lié à la cage 10, au moyen d'un amorçoir 510, il réalise, dans le corps osseux de chacune des deux vertèbres, un avant-trou d'une section inférieure à celle de la tige 21. Dans ce but l'amorçoir 510 est tour à tour engagé dans les canons de guidage 501.

Ces deux avant-trous réalisés, il peut, toujours par référence à cet ancillaire, mettre en place un premier pion d'ancrage 20 par l'intermédiaire de cet ancillaire 500 et d'un positionneur 520 apte, en vue de la mise en place du pion 20, à être introduit dans le canon de guidage 501 correspondant.

Ce positionneur 520 est avantageusement constitué d'un corps tubulaire 521 comportant une zone distale formant une chambre 522 prévue pour recevoir le pion à fixer. Une ouverture radiale 523 est pratiquée dans le corps tubulaire 521 pour l'introduction du pion 20 dans la chambre 522.

Le positionneur 520 est de plus doté d'un poussoir 524, sous forme de tige cylindrique, monté en coulissement dans le corps tubulaire, doté extérieurement à ce dernier d'un bouton de manoeuvre 525. Ce poussoir est apte, par déplacement dans le corps tubulaire 521 à venir pousser le pion et le chasser hors de la chambre 522 et hors du corps tubulaire 521.

Avantageusement, le corps tubulaire, en extrémité distale est doté d'un lamage 527 prévu pour être engagé autour d'un des canons de guidage 501. Une telle disposition assure un positionnement précis du positionneur 520 par rapport au canon et par voie de conséquence par rapport à la cage 10.

La zone distale du corps du positionneur est dotée d'une fente diamétrale 528 qui confère à cette zone une fonction de pince. On peut voir que cette fente diamétrale traverse de part en part la chambre 522 et prend naissance en amont de la chambre et court jusqu'à l'extrémité distale du positionneur. Ainsi le pion 20 est maintenu en place dans le corps tubulaire 521 par de légères forces radiales de serrage suffisantes pour assurer son maintien dans le corps et éviter sa chute mais suffisamment faibles pour écarter tous risques de détérioration des formes saillantes en sapine 25, 25a lors de son coulissement vers l'extrémité distale du corps tubulaire. En combinaison avec cette caractéristique, le poussoir 524 est sollicité vers la chambre par un organe élastique 526 formé par un ressort à spires. Ce ressort à spires est monté dans le corps 521 autour du poussoir. En raison de ces dispositions, le pion 20 est maintenu dans la chambre tant par les forces radiales de serrage que par l'action axiale du poussoir, étant entendu que cette action axiale, dont l'intensité dépend du degré de compression du ressort à spires, est insuffisante à elle seule pour pousser le pion hors de la chambre.

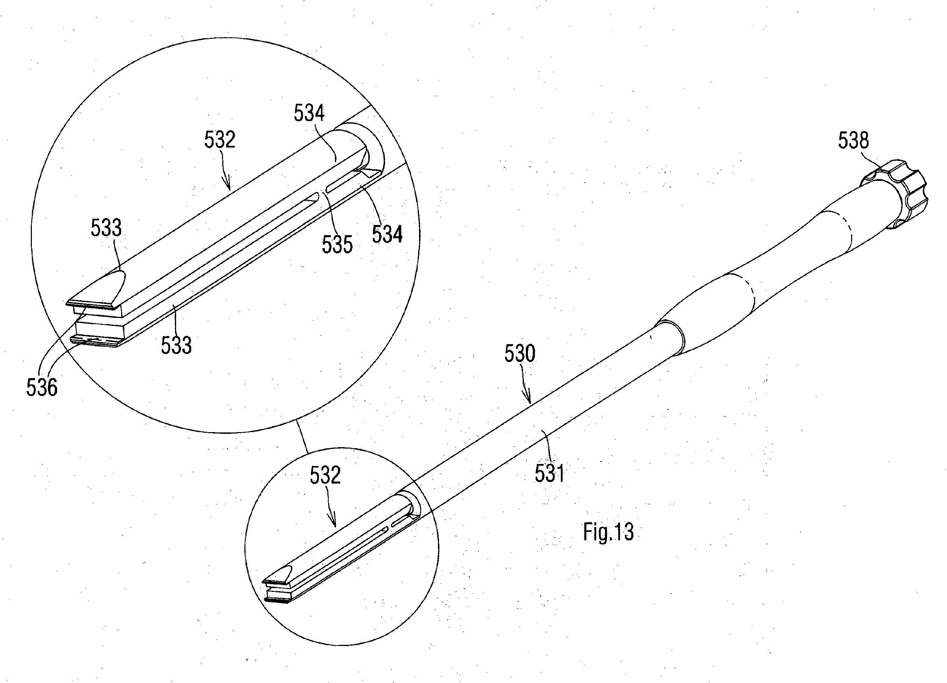

En vue de de la fixation de chaque pion 20 dans le corps vertébral correspondant, le positionneur 520, par son lamage 527 est positionné sur le canon de perçage et le poussoir 524 est actionné en vue de chasser le pion 20 vers le logement 40 de la cage 10 et vers le corps vertébral. Une force est appliquée ensuite à l'aide d'un maillet sur le bouton 525 afin de finaliser la mise en place du pion dans le corps vertébral. Il y a lieu de noter que le déplacement axial du poussoir se trouve limité par le bouton de manoeuvre 525. Ce déplacement est suffisamment pour que la forme en sapine 24a que possède le pion puisse être amenée en regard de l'orifice de sortie du logement 40 correspondant, extérieurement à ce dernier.
Après mise en place des pions 20 dans les corps vertébraux, l'ancillaire 500 est retiré et la plaque antérieure 70 est placée sur la portion antérieure 13a de paroi latérale de la cage à l'aide d'un ancillaire de pose 530 tel que celui représenté en figure 13.

Selon la forme préférée de réalisation, cet ancillaire de pose comprend sur un même bâti longiforme 531, une pince 532 dotée de deux mâchoires 533 de préhension de la plaque antérieure, articulées l'une à l'autre et associées chacune à un levier de manoeuvre 534. Selon la forme préférée de réalisation, l'extrémité de chaque mâchoire 533 est agencée en feuillure 536 tant pour faciliter la préhension de la plaque antérieure 70 par ses bordures longitudinales que la solidarisation de la plaque antérieure à cage 10. On peut remarquer également que l'articulation des deux mâchoires 533 est formée par une cloison transversale 535 élastique, enracinée aux deux mâchoires 533. Avantageusement, l'une des deux mâchoires 533 et le levier associé 534 sont formés par la partie distale du bâti 531. Le bâti porte de plus un moyen de manoeuvre des mâchoires 533 agissant sur les leviers 534. On peut remarquer que la distance entre la cloison d'articulation 535 et le point d'application des efforts par le moyen de manoeuvre sur les leviers 534 est moindre que la distance entre la cloison d'articulation et les feuillures d'extrémité 536. On réalise ainsi une démultiplication des efforts, au niveau des mâchoires de préhension de la plaque, tout en exerçant un effort moindre au niveau du levier 534. Cette disposition permet d'assurer la préhension, de la plaque, dans de bonnes conditions, et de garantir sa tenue, sur l'instrument, durant les phases de manipulation avant mise en place.

Dans la forme préférée de réalisation, le moyen de manoeuvre est formé d'une tige537 montée en vissage dans le corps du bâti, ledit corps étant tubulaire. L'extrémité proximale de la tige reçoit un bouton 538 de manoeuvre en rotation. L'extrémité distale de la tige, qui présente une forme sphérique, s'engage entre les leviers 534 pour agir radialement sur ces derniers et les écarter angulairement l'un de l'autre. Est ainsi réalisé le serrage des mâchoires 533 sur la plaque antérieure 70.

Selon la forme préférée de réalisation, la tige 537 à proximité de son extrémité proximale comporte un collet fileté 539 engagé en vissage dans un taraudage traversant formé dans la cloison terminale 540 que le corps tubulaire 531 comporte à son extrémité proximale. A distance de ce premier collet 539, la tige 537 comporte un second collet 541 également fileté. Une telle disposition, dans le cas d'un dévissage total du premier collet 539, s'oppose à la perte de la tige, le second collet 541 venant en butée contre la cloison terminale 540. Mais cette disposition autorise cependant le démontage de la tige, le second collet, pour ce faire, étant alors engagé en vissage dans le taraudage de la cloison terminale.

## Revendications

1. Système pour réaliser une arthrodèse entre deux première et seconde vertèbres consécutives (V1, V2), plus particulièrement des vertèbres cervicales, comportant :
▪ cage (10) apte à être interposée entre les deux dites vertèbres en remplacement du disque intervertébral qui était originellement situé entre ces deux vertèbres, ladite cage étant définie dans une enveloppe parallélépipédique rectangle, ladite cage comportant deux première et seconde faces d'appui, opposées (11, 12) aptes à venir au contact respectivement des deux corps osseux des deux dites vertèbres (V1, V2) quand la cage (10) est interposée entre elles et une paroi latérale (13) réunissant les deux dites faces opposées (11, 12, ladite paroi latérale étant composée de quatre portions de paroi à savoir une portion antérieure (13a) et une portion postérieure (13b) normales au plan sagittal et deux portions (13c) gauche et droite), et
▪ des moyens pour stabiliser ladite cage avec le corps osseux d'au moins l'une première (VI) des deux vertèbres consécutives, ces dits moyens étant des moyens de stabilisation postéro-antérieur et étant constitués par deux ensembles (E1, E2) comportant chacun au moins:
- un élément d'ancrage (20), sous forme de pion comportant :
∘ une tige (21) définie selon un premier axe longitudinal (121) entre une extrémité proximale (22) et une extrémité distale (23), l'extrémité distale étant conformée en extrémité pénétrante, ladite tige étant apte à être ancrée dans le corps osseux de la première vertèbre en pénétrant, dans ce corps osseux, par son extrémité distale pénétrante,
∘ une tête cylindrique (30) ayant une longueur déterminée définie selon un deuxième axe longitudinal (131), la dite tête étant solidaire de ladite tige et étant formée en extrémité proximale de cette dernière,
et un logement (40) de forme sensiblement cylindrique défini selon un troisième axe longitudinal (143) et réalisé dans ladite cage (10) de façon qu'il débouche sur ladite paroi latérale (13) par un orifice d'entrée (44) et sur la première face d'appui par un orifice de sortie (45), ledit troisième axe longitudinal (143) faisant un angle déterminé par rapport au plan passant sensiblement par la deuxième face (12) d'appui, ce logement (40) étant doté d'une fente radiale (46) s'étendant d'un orifice à l'autre et débouchant sur la première face d'appui (11), et ledit logement étant en outre réalisé de façon que, lorsque ladite tête cylindrique (30) est enfichée dans le logement (40), le premier axe longitudinal (121) de la tige fasse avec le plan de la seconde face (12) un angle (β) non nul et que, lorsque ladite cage (10) est interposée entre les deux vertèbres consécutives (V1, V2), ladite tige (21) déborde du logement par l'orifice de sortie (45) et ladite tête (30) par la fente radiale (46) et pénètrent toutes deux dans le corps osseux de la première vertèbre (V1) qui est au contact de cette première face (11),
ledit système comportant en outre une plaque antérieure (70) venant en recouvrement partiel au moins de l'entrée (44) du logement (40) pour empêcher la sortie de la tête cylindrique (30) hors de chaque logement (40) après qu'elle y ait été enfichée, et des moyens pour solidariser cette plaque antérieure avec la cage (10), lesquels sont constitués d'une part, par deux pattes latérales (71) que comporte ladite plaque, ces pattes latérales (71) présentant en extrémité des bossages de rétention (72) et d'autre part par des formes en creux (13d) complémentaires de celles des bossages et prévues pour recevoir ces derniers, ces formes en creux (13d) étant ménagées dans les parois (13c) gauche et droite de la cage (10),
**caractérisé en ce que** les arêtes de la plaque antérieure (70), des pattes latérales (71) et des bossages (72) sont arrondies, que l'extrémité libre de chaque patte latérale (71) est arrondie, que chaque bossage (72) est cylindrique, que ladite plaque (70) vient en recouvrement partiel des deux entrées (44) des deux logements (40) respectivement des deux ensembles (E1, E2) et que la plaque antérieure (70), est montée sur la cage avec une aptitude de pivotement limité autour de l'axe géométrique défini par les bossages (72) et les formes en creux (13d), cet axe géométrique de pivotement étant parallèle à la face antérieure de la portion de paroi (13a) afin que la migration vers la plaque de l'un des pions, se traduise par un effort de poussée de ce pion par sa face terminale antérieure, contre le bord longitudinal correspondant de la face interne de la plaque antérieure (70), et par voie de conséquence par le basculement de cette plaque antérieure (70) vers l'autre pion et plus particulièrement vers l'entrée (44) du logement (40) accueillant cet autre pion.

2. Système selon la revendication 1, **caractérisé par le fait que** les premier et deuxième axes longitudinaux (121, 131) sont confondus.

3. Système selon les revendications 1 ou 2, **caractérisé par le fait que** le logement (40) a une profondeur au moins égale à la longueur de ladite tête cylindrique (30) de façon que ladite tête cylindrique soit apte à y pénétrer entièrement.

4. Système selon la revendication précédente, **caractérisé en ce que** le logement (40) présente un épaulement interne (47) contre lequel vient en appui une surface de butée 31, annulaire de la tête lorsque l'élément d'ancrage est en place par rapport à la cage.

5. Système selon l'une quelconque des revendications précédentes, **caractérisé en ce que** la tige (21) et la tête (30) présentent des formes en sapine (25, 25a) sous forme de collerette annulaire, agencées pour autoriser l'introduction de la tige et de la tête dans le corps osseux et pour s'opposer à leur extraction.

6. Système selon la revendication précédente, **caractérisé en ce que** celle des formes en sapine (25a) de la tige (21), prévue pour être située immédiatement en regard de l'orifice de sortie (45) du logement (40) présente un diamètre supérieur au diamètre dudit orifice.

7. Système selon l'une quelconque des revendications précédentes, **caractérisé par le fait que** ledit troisième axe longitudinal (143) fait, avec le plan de la seconde face (12), un angle égal au dit angle non nul (β).

8. Système selon l'une quelconque des revendications précédentes, **caractérisé en ce que** les parois gauche et droite (13c) présentent chacune une gorge (13e) apte à recevoir la patte correspondante (71) de la plaque antérieure (70), que chaque gorge (13e) débouche radialement dans la forme en creux (13d) correspondante et que la profondeur de chaque gorge (13e) est égale ou supérieure à l'épaisseur de chaque patte latérale (71) afin qu'après mise en place de la plaque antérieure (70), les pattes latérales (71) ne soient pas en débordement par rapport aux faces externes à la cage des parois (13c).

9. Système selon la revendication 8, **caractérisé en ce que** chaque gorge (13e) est de forme évasée et s'élargit progressivement depuis la forme en creux (13d) correspondante vers la face antérieure de la portion de paroi (13a), que le fond plat de chaque gorge (13e) est incliné latéralement vers l'extérieur depuis la face antérieure de la portion de paroi (13a), vers la forme en creux (13d) et que la face terminale de chaque bossage (72) est oblique par rapport à l'axe géométrique de révolution que possède ce bossage, et est parallèle lors de la mise en place de la plaque antérieure au fond plat de la gorge correspondante.

10. Système selon l'une quelconque des revendications précédentes, **caractérisé par le fait qu'**il comporte les deux logements (40) respectivement des deux ensembles sont réalisés de façon que, lorsque la tête cylindrique (30) de chaque ensemble est enfichée dans l'orifice correspondant, les premiers axes longitudinaux (121) des deux tiges (20) fassent respectivement avec le plan.de la seconde face (12) un angle (β) non nul et que, lorsque ladite cage (10) est interposée entre les deux vertèbres, les tiges (21) pénètrent respectivement dans le corps osseux des deux vertèbres (VI; V2) respectivement au contact des première et seconde faces (11, 12) de la cage (10).

11. Positionneur (520) pour la mise en place dans un corps vertébral d'un pion (20) d'un système selon l'une quelconque des revendications précédentes, **caractérisé en ce qu'**il comprend d'un corps tubulaire (521) comportant une zone distale formant une chambre (522) prévue pour recevoir le pion à introduire dans le corps vertébral, ladite chambre (522) étant dotée d'une ouverture radiale (523) d'introduction du pion (20), ledit positionneur comportant un poussoir (524) sous forme de tige cylindrique, monté en coulissement dans le corps tubulaire, doté extérieurement à ce dernier d'un bouton de manoeuvre (525).

12. Positionneur (520) selon la revendication précédente, **caractérisé en ce que** la zone distale du corps tubulaire (521) est dotée d'une fente diamétrale (528) conférant à cette zone une fonction de pince et que le poussoir (524) est sollicité vers la chambre par un organe élastique (526).

## Patentansprüche

1. System zur Durchführung einer Arthrodese zwischen zwei aufeinanderfolgenden Wirbeln, einem ersten und einem zweiten (V1, V2), insbesondere Halswirbeln, mit
▪ einem Gehäuse (10), das zwischen die zwei genannten Wirbel als Ersatz für die Bandscheibe einsetzbar ist, die sich ursprünglich zwischen diesen beiden Wirbeln befand, wobei das Gehäuse in einer rechteckigen parallelepipeden Umhüllung ausgebildet ist, wobei das Gehäuse eine erste und eine zweite Anlagefläche (11, 12), die einander gegenüberliegen und in der Lage sind, jeweils mit den beiden knöchernen Körpern der beiden genannten Wirbel (V1, V2) in Kontakt zu kommen, wenn das Gehäuse (10) zwischen sie eingesetzt ist, und eine Seitenwand (13) aufweist, die die beiden gegenüberliegenden Flächen (11, 12) vereint, wobei die Seitenwand aus vier Wandabschnitten besteht, nämlich einem vorderen Abschnitt (13a) und einem hinteren Abschnitt (13b), die normal zur Sagittalebene sind, und einem linken und einem rechten Abschnitt (13c),
▪ Einrichtungen zum Stabilisieren des Gehäuses mit dem knöchernen Körper wenigstens eines ersten (V1) der beiden aufeinanderfolgenden Wirbel, wobei die genannten Einrichtungen Einrichtungen zur Stabilisation nach hinten und vorne sind und aus zwei Einheiten (E1, E2) bestehen, die jeweils wenigstens umfassen:
- ein Verankerungselement (20) in Form eines Bolzens mit
∘ einem Stift (21), der gemäß einer ersten Längsachse (121) zwischen einem proximalen Ende (22) und einem distalen Ende (23) ausgebildet ist, wobei das distale Ende als eindringendes Ende angepasst ist, wobei der Stift in dem knöchernen Körper des ersten Wirbels verankerbar ist, indem er mit seinem eindringenden distalen Ende in den knöchernen Körper eindringt,
∘ einem zylindrischen Kopf (30), der eine bestimmte Länge hat, die gemäß einer zweiten Längsachse (131) definiert ist, wobei der Kopf mit dem Stift fest verbunden ist und als proximales Ende des Letzteren ausgebildet ist,
- und eine Aufnahme (40) von im Wesentlichen zylindrischer Form, die gemäß einer dritten Längsachse (143) definiert und in dem Gehäuse (10) derart ausgebildet ist, dass sie an der Seitenwand (13) mit einer Eingangsöffnung (44) und an der ersten Anlagefläche mit einer Ausgangsöffnung (45) mündet, wobei die dritte Längsachse (143) einen Winkel bildet, der bezogen auf die Ebene bestimmt ist, die im Wesentlichen durch die zweite Anlagefläche (12) verläuft, wobei die Aufnahme (40) mit einer radialen Aussparung (46) versehen ist, die sich von einer Öffnung zur anderen erstreckt und an der ersten Anlagefläche (11) mündet, und wobei die Aufnahme außerdem derart ausgebildet ist, dass, wenn der zylindrische Kopf (30) in die Aufnahme (40) eingesteckt ist, die erste Längsachse (121) des Stifts mit der Ebene der zweiten Fläche (12) einen Winkel (β) bildet, der nicht null ist, und dass, wenn das Gehäuse (10) zwischen die zwei aufeinanderfolgenden Wirbel (V1, V2) eingesetzt ist, der Stift (21) aus der Aufnahme durch die Ausgangsöffnung (45) und der Kopf (30) durch die radiale Aussparung (46) vorsteht und alle beide in den knöchernen Körper des ersten Wirbels (V1) eindringen, der mit dieser ersten Fläche (11) in Kontakt steht,
wobei das System weiterhin eine vordere Platte (70), die wenigstens mit dem Eingang (44) der Aufnahme (40) in eine teilweise Überdeckung kommt, um das Austreten des zylindrischen Kopfs (30) aus jeder Aufnahme (40) nach seinem Hineinstecken zu verhindern, und einstückige Einrichtungen zum Befestigen dieser vorderen Platte an dem Gehäuse (10) durch zwei seitliche Laschen (71) aufweist, die die Platte aufweist, wobei die seitlichen Laschen (71) an ihrem Ende Rückhaltevorsprünge (72) aufweisen, und andererseits durch Hohlformen (13d), die zu denen der Vorsprünge komplementär sind und dazu vorgesehen sind, Letztere aufzunehmen, wobei diese Hohlformen (13d) in der linken und rechten Wand (13c) des Gehäuses (10) angeordnet sind, **dadurch gekennzeichnet, dass** die Kanten der vorderen Platte (70), der seitlichen Laschen (71) und der Vorsprünge (72) abgerundet sind, dass das freie Ende jeder seitlichen Lasche (71) abgerundet ist, dass jeder Vorsprung (72) zylindrisch ist, dass die Platte (70) jeweils mit den beiden Eingängen (44) der beiden Aufnahmen (40) der zwei Einheiten (E1, E2) in teilweise Überdeckung kommt und dass die vordere Platte (70) an dem Gehäuse mit einer begrenzten Schwenkfähigkeit um die durch die Vorsprünge (72) und die Hohlformen (13d) definierte geometrische Achse montiert ist, wobei diese geometrische Schwenkachse zur Vorderfläche des Wandabschnitts (13a) parallel ist, damit sich die Wanderung eines der Bolzen zur Platte in einer Schubwirkung dieses Bolzens über seine vordere Stirnfläche gegen den Längsrand, der der Innenfläche der vorderen Platte (70) entspricht, und folglich einem Kippen dieser vorderen Platte (70) zum anderen Bolzen und insbesondere zum Eingang (44) der Aufnahme (40), die den anderen Bolzen aufnimmt, niederschlägt.

2. System nach Anspruch 1, **dadurch gekennzeichnet, dass** die erste und die zweite Längsachse (121, 131) zusammenfallen.

3. System nach den Ansprüchen 1 oder 2, **dadurch gekennzeichnet, dass** die Aufnahme (40) eine Tiefe hat, die wenigstens gleich der Länge des zylindrischen Kopfes (30) ist, derart, dass der zylindrische Kopf in der Lage ist, vollständig in sie einzudringen.

4. System nach dem vorhergehenden Anspruch, **dadurch gekennzeichnet, dass** die Aufnahme (40) eine Innenschulter (47) aufweist, gegen die eine ringförmige Anschlagfläche (31) des Kopfs anliegt, wenn das Verankerungselement bezogen auf das Gehäuse positioniert ist.

5. System nach irgendeinem der vorhergehenden Ansprüche, **dadurch gekennzeichnet, dass** der Stift (21) und der Kopf (30) Balkenformen (25, 25a) in Form eines ringförmigen Kragens aufweisen, die dazu ausgelegt sind, das Einführen des Stiftes und des Kopfes in den knöchernen Körper zu erlauben und sich ihrem Herausziehen zu widersetzen.

6. System nach dem vorhergehenden Anspruch, **dadurch gekennzeichnet, dass** diejenige Balkenform (25a) des Stiftes (21), die dafür vorgesehen ist, sich unmittelbar gegenüber der Ausgangsöffnung (45) der Aufnahme (40) zu befinden, einen Durchmesser aufweist, der größer ist als der Durchmesser der Öffnung.

7. System nach irgendeinem der vorhergehenden Ansprüche, **dadurch gekennzeichnet, dass** die dritte Längsachse (143) mit der Ebene der zweiten Fläche (12) einen Winkel bildet, der gleich dem genannten Winkel (β) ist, der nicht null ist.

8. System nach irgendeinem der vorhergehenden Ansprüche, **dadurch gekennzeichnet, dass** die linke und die rechte Wand (13c) jeweils eine Aussparung (13e) aufweist, die in der Lage ist, die entsprechende Lasche (71) der vorderen Platte (70) aufzunehmen, dass jede Aussparung (13e) radial in der entsprechenden Hohlform (13d) mündet und dass die Tiefe jeder Aussparung (13e) gleich oder größer als die Dicke jeder Seitenlasche (71) ist, damit die seitlichen Laschen (71) nach der Positionierung der vorderen Platte (70) bezogen auf die außerhalb des Gehäuses liegenden Flächen der Wände (13c) nicht vorstehen.

9. System nach Anspruch 8, **dadurch gekennzeichnet, dass** jede Aussparung (13e) eine sich erweiternde Form hat und sich von der entsprechenden Hohlform (13d) zur vorderen Fläche des Abschnitts der Wand (13a) progressiv erweitert, dass der ebene Boden jeder Aussparung (13e) ausgehend von der vorderen Fläche des Abschnitts der Wand (13a) zur Hohlform (13d) seitlich nach außen geneigt ist, und dass die Stirnfläche jedes Vorsprungs (72), bezogen auf die geometrische Umdrehungsachse, die dieser Vorsprung besitzt, schräg ist, und bei Positionierung der vorderen Wand am ebenen Boden der entsprechenden Aussparung parallel ist.

10. System nach irgendeinem der vorhergehenden Ansprüche, **dadurch gekennzeichnet, dass** es jeweils die zwei Aufnahmen (40) der zwei Einheiten aufweist, die derart ausgeführt sind, dass, wenn der zylindrische Kopf (30) jeder Einheit in die entsprechende Öffnung eingesteckt ist, die ersten Längsachsen (121) der zwei Stifte (21) jeweils mit der Ebene der zweiten Fläche (12) einen Winkel (β) bilden, der nicht null ist, und dass wenn das Gehäuse (10) zwischen die zwei Wirbel eingesetzt ist, die Stifte (21) jeweils in den knöchernen Körper der zwei Wirbel (V1, V2) in Kontakt mit der ersten und der zweiten Fläche (11, 12) des Gehäuses (10) eindringen.

11. Positioniervorrichtung (520) zum Positionieren eines Bolzens (20) eines Systems nach irgendeinem der vorhergehenden Ansprüche in einem Wirbelkörper, **dadurch gekennzeichnet, dass** sie einen rohrförmigen Körper (521) aufweist, der einen distalen Bereich hat, der eine Kammer (522) bildet, die für die Aufnahme des in den Wirbelkörper einzuführenden Bolzens vorgesehen ist, wobei die Kammer (522) mit einer radialen Öffnung (523) für das Einführen des Bolzens (20) versehen ist, wobei die Positioniervorrichtung einen Stößel (524) in Form eines zylindrischen Stiftes aufweist, der verschiebbar in dem rohrförmigen Körper montiert ist, der außerhalb von Letzterem mit einem Betätigungsknopf (525) versehen ist.

12. Positioniervorrichtung (520) nach dem vorhergehenden Anspruch, **dadurch gekennzeichnet, dass** der distale Bereich des rohrförmigen Körpers (521) mit einer diametralen Aussparung (528) versehen ist, die diesem Bereich eine Klemmfunktion verleiht, und dass der Stößel (524) zur Kammer hin durch eine elastische Einrichtung (526) vorgespannt ist.

## Claims

1. System for performing arthrodesis between two consecutive first and second vertebrae (V1, V2), more particularly cervical vertebrae, said system having:
• a cage (10) that can be interposed between said two vertebrae to replace the intervertebral disc that was originally situated between these two vertebrae, said cage being defined within a rectangular parallelepipedal envelope, said cage having two opposite first and second bearing faces (11, 12) that can come into contact with the two osseous bodies of said two vertebrae (V1, V2), respectively, when the cage (10) is interposed between these, and a lateral wall (13) joining said two opposite faces (11, 12), said lateral wall being composed of four wall portions, namely an anterior portion (13a) and a posterior portion (13b), which are perpendicular to the sagittal plane, and two left and right portions (13c), and
• means for stabilizing said cage with the osseous body of at least a first (V1) of the two consecutive vertebrae, said means being means for posterior-anterior stabilization and being formed by two assemblies (E1, E2) that each have at least:
- an anchoring element (20), in the form of a peg, having:
• a rod (21) defined along a first longitudinal axis (121) between a proximal end (22) and a distal end (23), the distal end being configured as a penetrating end, said rod being able to be anchored in the osseous body of the first vertebra by penetrating into this osseous body with its penetrating distal end,
• a cylindrical head (30) having a specific length defined along a second longitudinal axis (131), said head being rigidly connected to said rod and being formed as the proximal end of the latter,
- and a seat (40) of substantially cylindrical shape defined along a third longitudinal axis (143) and formed in said cage (10) in such a way that it opens out on said lateral wall (13) via an inlet orifice (44) and on the first bearing face via an outlet orifice (45), said third longitudinal axis (143) forming a defined angle with respect to the plane passing substantially through the second bearing face (12), this seat (40) being provided with a radial slit (46) extending from one orifice to the other and opening out on the first bearing face (11), and said seat additionally being formed in such a way that, when said cylindrical head (30) is engaged in the seat (40), the first longitudinal axis (121) of the rod forms, with the plane of the second face (12), a non-zero angle (β), and, when said cage (10) is interposed between the two consecutive vertebrae (V1, V2), said rod (21) protrudes from the seat through the outlet orifice (45) and said head (30) protrudes through the radial slit (46) and both of them penetrate into the osseous body of the first vertebra (V1) that is in contact with this first face (11),
said system additionally having an anterior plate (70) that comes to partially cover at least the inlet (44) of the seat (40) in order to prevent the cylindrical head (30) from leaving each seat (40) after it has been engaged therein, and means for rigidly connecting this anterior plate to the cage (10), which means are formed, on the one hand, by two lateral tabs (71) arranged on said plate, these lateral tabs (71) having retention bosses (72) at their ends, and, on the other hand, by recessed formations (13d) complementing the bosses and designed to receive the latter, these recessed formations (13d) being formed in the left and right walls (13c) of the cage (10),
**characterized in that** the edges of the anterior plate (70), of the lateral tabs (71) and of the bosses (72) are rounded, **in that** the free end of each lateral tab (71) is rounded, **in that** each boss (72) is cylindrical, **in that** said plate (70) comes to partially cover the two inlets (44) of the two seats (40), respectively, of the two assemblies (E1, E2), and **in that** the anterior plate (70) is mounted on the cage with a capacity for limited pivoting about the geometric axis defined by the bosses (72) and the recessed formations (13d), this geometric axis of pivoting being parallel to the anterior face of the wall portion (13a), such that the migration of one of the pegs towards the plate translates into a thrusting force exerted by the anterior end face of this peg against the corresponding longitudinal edge of the inner face of the anterior plate (70), and, consequently, into the tilting of this anterior plate (70) towards the other peg and more particularly towards the inlet (44) of the seat (40) that receives this other peg.

2. System according to Claim 1, **characterized in that** the first and second longitudinal axes (121, 131) are coincident.

3. System according to Claim 1 or 2, **characterized in that** the seat (40) has a depth at least equal to the length of said cylindrical head (30), such that said cylindrical head is able to penetrate fully therein.

4. System according to the preceding claim, **characterized in that** the seat (40) has an inner shoulder (47) against which an annular abutment surface (31) of the head bears when the anchoring element is in place with respect to the cage.

5. System according to any one of the preceding claims, **characterized in that** the rod (21) and the head (30) have fir-tree shapes (25, 25a), in the form of an annular collar, which are designed to permit the introduction of the rod and of the head into the osseous body and to oppose their removal from same.

6. System according to the preceding claim, **characterized in that** the fir-tree shape (25a) of the rod (21) intended to be situated immediately opposite the outlet orifice (45) of the seat (40) has a diameter greater than the diameter of said orifice.

7. System according to any one of the preceding claims, **characterized in that** said third longitudinal axis (143) forms, with the plane of the second face (12), an angle equal to said non-zero angle (β).

8. System according to any one of the preceding claims, **characterized in that** the left and right walls (13c) each have a groove (13e) that is able to receive the corresponding tab (71) of the anterior plate (70), **in that** each groove (13e) opens out radially in the corresponding recessed formation (13d), and **in that** the depth of each groove (13e) is equal to or greater than the thickness of each lateral tab (71) such that, after the anterior plate (70) has been fitted in place, the lateral tabs (71) do not protrude, with respect to the outer faces of the cage, from the walls (13c).

9. System according to Claim 8, **characterized in that** each groove (13e) has a flared shape and widens progressively from the corresponding recessed formation (13d) towards the anterior face of the wall portion (13a), **in that** the flat bottom of each groove (13e) is inclined laterally outwards from the anterior face of the wall portion (13a) towards the recessed formation (13d), and **in that** the end face of each boss (72) is oblique with respect to the geometric axis of revolution of this boss and is parallel, when the anterior plate is fitted in place, to the flat bottom of the corresponding groove.

10. System according to any one of the preceding claims, **characterized in that** the two seats (40) of the two assemblies are formed in such a way that, when the cylindrical head (30) of each assembly is engaged in the corresponding orifice, the first longitudinal axes (121) of the two rods (21) form respectively with the plane of the second face (12) a non-zero angle (β) and, when said cage (10) is interposed between the two vertebrae, the rods (21) penetrate respectively into the osseous bodies of the two vertebrae (V1, V2) which are respectively in contact with the first and second faces (11, 12) of the cage (10).

11. Positioner (520) by which a peg (20) of a system according to any one of the preceding claims is placed in a vertebral body, **characterized in that** it comprises a tubular body (521) having a distal zone that forms a chamber (522) intended to receive the peg to be introduced into the vertebral body, said chamber (522) being provided with a radial opening (523) for introduction of the peg (20), said positioner having a pusher (524) in the form of a cylindrical rod which is mounted so as to slide in the tubular body and is equipped externally with a control button (525).

12. Positioner (520) according to the preceding claim, **characterized in that** the distal zone of the tubular body (521) is provided with a diametral slit (528) that gives this zone a gripping function, and **in that** the pusher (524) is stressed towards the chamber by an elastic member (526).
